# EUROPEAN PATENT APPLICATION

(11) **EP 1 391 151 A1**
(43) Date of publication of application: **25.02.2004**
(21) Application number: 02447156.7
(22) Date of filing: 19.08.2002
(51) Int. Cl.: A01N 1/02

(54) **Thinner for conservation of sperm**

(71) Applicant: Stoeterij Zangersheide N.V., 3620 Lanaken (BE)
(72) Inventor: De Backer, Leo, 2950 Kapellen (BE); Poncelet, Steven, 3590 Diepenbeek (BE)
(74) Representative: De Clercq, Ann

(57) **Abstract**

The present invention relates to a novel thinner for the conservation of fresh sperm in liquid or powder form. The invention preferably relates to the conservation of fresh horse sperm. The invention further relates to the uses of said compositions, methods for preparing said thinners, methods for preparing sperm solutions comprising the same, vials comprising the same as well as their preparation method, as well as methods to preserve sperm or fertilize oocytes based thereon.

## Description

### FIELD OF THE INVENTION

The proposed invention is based on the protective effect of milk proteins for the improvement of the viability of sperm cells and their disposition with respect to fertility, in anticipation of artificial insemination.

### BACKGROUND OF THE INVENTION

With the techniques currently used for artificial insemination, the conservation of sperm in anticipation of the insemination process presents a problem.

After collection, the sperm very quickly loses its capacity for fertilisation. In order to remedy this situation, thus to have the possibility to conserve the sperm until the moment of artificial insemination, one needs to have recourse to an adapted environment and a diluting agent as quickly as possible following the collecting of the sperm.

The duration for conservation that can be achieved depends on the thinner used and on the conservation conditions. In general, the conservation period may fluctuate between a few hours and a few days. For some species, it is possible to prolong the conservation term by deep-freezing the sperm but this technique may also result in a destruction or inactivity of the sperm and thus result in a lowered fertility.

Other diluting agents or thinners are known for the conservation of fresh sperm; every one of these thinners contains nutrients, a buffer, and mineral salts.

The thinners ensure a better and more comfortable conservation and maintain the fertility of the sperm.

Amongst the thinning agents that are best known for the conservation of sperm of different species of animals, for instance sheep, cattle, pigs and horses, we often find milk or thinners based on milk.

Temperature also plays a big role in the conservation. The metabolic processes proceed completely differently at 5°C than at 20°C. While there have been various studies on this subject, using different thinners, the results are quite divergent. For instance, Varner et al. [Theriogenology, (32)515-525, (1989)] used KENNEY thinner and finds no difference whatsoever in the fertility with the use of sperm kept at 5°C and at 20°C; Squires et al. [Proc 11^{th} International Congress on Animal Reproduction and Al, Dublin, Ireland, (3)297, (1988)] use the same thinner and achieve a better fertility with sperm stored at 5°C, but only in the case of certain studs.

As most carriers and veterinarians have their services geared to the conservation of fresh sperm at a temperature of 5°C, the present invention aims to develop diluting agents on that basis.

The duration of the conservation life has also become a subject of controversy. Certain authors [Douglas-Hamilton et al., Theriogenology, (22) 291-304, (1984)] are using doses 6 to 24 hours after collection and are getting in-foal mares as a result. Others [Squires et al., Proc. 11^{th} International congress on Animal Reproduction and Al, Dublin, Ireland, (3) 297, (1988)] do not achieve any kind of pregnancy with sperm that was preserved for 48 hours at 20° C. A more recent study [Heiskanten et al., Theriogenology (42) 1043-1051 (1994)] uses sperm that was stored for 70-80 hours at 5° C in the supplemented Kenney thinner and obtained gestation percentages between 57% and 77% per cycle.

### SUMMARY OF THE INVENTION

The invention relates to a liquid composition comprising per liter:

| | |
|---|---|
| Glucose monohydrate | 0.1-50 g ; |
| Lactose monohydrate | 0.1-50 g , |
| Sodium citrate dihydrate | 0.1-1 g , |
| Potassium citrate | 0.1-1 g , |
| Hepes | 0.1-10 g , |
| Non-fat powdered milk | 40-60 g , |
| optionally, antibiotics | 10-100 mg , and, |
| water. | |

The amount of Glucose monohydrate may be 0.5, 1, 3, 12, 17, 21, 33, 40, 45 or 50 g.
The amount of Lactose monohydrate may be 0.5, 1, 3, 12, 17, 21, 33, 40, 45 or 50 g.
The amount of Sodium citrate dihydrate may be 0.2, 0.3, 0.4, 0.5, 0.6, 0.7, 0.8, 0.9 or 1 g.
The amount of Potassium citrate may be 0.2, 0.3, 0.4, 0.5, 0.6, 0.7, 0.8, 0.9 or 1 g.
The amount of Hepes may be 0.3, 0.5, 1, 1.2, 2.3, 3.5, 4.9, 5.1, 7.5 or 10 g.
The amount of Non-fat powdered milk may be 45, 47, 53, 55, 56, 58 or 60 g
The optional amount of antibiotics may be 15, 23, 37, 42, 56, 78, 92 or 100 mg.

According to a specific embodiment the invention relates to a liquid composition comprising per liter:

| | |
|---|---|
| Glucose monohydrate | 27.2 g ; |
| Lactose monohydrate | 1.5 g , |
| Sodium citrate dihydrate | 0.25 g , |
| Potassium citrate | 0.41 g , |
| Hepes | 4.75 g , |
| Non-fat powdered milk | 50 g , |
| optionally, antibiotics | 50 mg , and, |
| water. | |

Said milk in said liquid composition can be Gloria® manufactured by Nestle®.

According to a further embodiment said water in said liquid composition is demineralised; preferably sterilized.

According to a further embodiment said antibiotic is Gentamycin. Gentamycin is an ideal antibiotic for use with living cultures, as it has a broad antibacterial spectrum and anti-mycoplasmal activity and is free from cell toxicity. It has biological and biochemical properties which render it superior to the use of Penicilin and Streptomycin. Normally it is used in concentrations of 25-50µg/ml. As well as being active against many gram positive or gram negative organisms, Gentamicin is active against strains of Proteus and Staphylococcus which can sometimes be resistant to a Penicillin/Streptomycin mix and unlike Pencilin and Streptomycin, Gentamicin is active against Pseudomonas strains. Gentamicin is stable over pH range of 2 to 10, thus ideal for the use in the extender. Gentamicin is stable at all temperatures and can withstand autoclaving. Gentamicin can thus be added before sterilisation of the solution, other antibiotics such as Penicillin and Streptomycin are inactivated or reduced by about 50% by autoclaving. Gentamicin is ideal for transport of clinical specimens in which overgrowth of bacteria should be avoided.

According to a further embodiment said liquid composition has a pH between 6.3 and 7.0. Possibly said pH is 6.3, 6.4, 6.5, 6.6, 6.7, 6.8, 6.9 or 7.0.

The invention also relates to a powder composition prepared by means of a drying procedure starting from a composition of any of claims 1 to 5.

According to a further embodiment said drying procedure is a freeze-drying procedure.

According to a further embodiment said powder composition is stored in the dark at temperatures between about 15 and 25°C for an unlimited time.

The invention also relates to a liquid composition prepared by dissolving a powder composition as defined above, using water; preferably demineralised and sterilized water.

According to a further embodiment said liquid composition as defined is stored at about 5°C Preferably this will be done for maximum 7 days.

According to a further embodiment said liquid composition as defined above is stabilized at room temperature for at least about 30 min, and thereafter heated to about 37°C just before use.

The invention also relates to a liquid composition as defined above, mixed with sperm.

According to a further embodiment said mixing is performed by swirling the recipient at the first stage of the processing of the sperm, after the doses are prepared, using a slowly rotating roller bank.

According to a further embodiment said sperm is of animal or human origin.

According to a further embodiment said animal is chosen from the group sheep, cattle, pig and horse.

According to a further embodiment said sperm is filtered before mixing it with the liquid composition as defined above.

According to a further embodiment said filtration of sperm is performed using a gauze.

According to a further embodiment said liquid thinner or extender composition as defined above (without sperm) is sterilized using an UHT method.

According to a further embodiment said sperm is diluted into the liquid composition as defined above reaching a sperm cell concentration between about 20-60x10⁶ cells/ml, preferably of 40x10⁶ cells/ml. Higher concentrations are also no problem, only the duration of the period in which they are to be kept becomes shorter since the nutrients will deteriorate quicker.

According to a further embodiment said sperm containing composition is used immediately to perform fertilization of oocytes, or, is conserved between about 2-10°C, preferably at about 5°C in anaerobic condition. By slowly cooling at a rate of 0.2 - 0.5 °C per minute.

The invention also relates to the use of a composition (liquid or powder) as a sperm thinner (sperm extender).

The invention also relates to the use of a composition as defined above, for the conservation of fresh sperm.

The invention also relates to the use of a composition as defined above, for the fertilization of oocytes.

The invention also relates to a method for the preparation of a thinner (extender) for the conservation of fresh sperm, comprising following steps:
a) preparing a liquid composition as defined above, and,
b) optionally, making powder of said composition for the long term conservation of said dried composition preferably using a freeze-drying procedure.

The invention also relates to a method for the preparation of a sperm solution for the fertilization of oocytes, comprising following steps:
a) preparing or obtaining a liquid composition as defined above,
b) optionally, making a powder of said composition for the long term conservation of said composition at room temperature for an unlimited time, preferably using a freeze-drying procedure, or obtaining said powder composition,
c) optionally, dissolving said powder using water, preferably demineralised or sterile water,
d) optionally, storing said liquid composition at about 4°C for maximum 7 days,
e) stabilizing said composition for about 30 min at 18 to 25°C,
f) warming up the composition to 37°C for about 15 min for 100 ml,
g) optionally, filtering the sperm, preferably using a gauze,
h) mixing the composition of step f) with the sperm, optionally of step g), preferably until a sperm cell concentration between about 20-60x10⁶ cells/ml, preferably of 40x10⁶ cells/ml is reached,
i) decreasing the temperature slowly at a rate of 0.2 -0.5°C per minute until about 5°C is reached, and,
j) conserving said composition at about 5°C in an anaerobic condition.

The mixing of the sperm with the composition of the invention is done at two times of the dose preparation. The first time the thinner is added after the filtration of the ejaculate and the second time it is done after centrifugation and removal of the supernatant. This time the sperm pellet is slightly stirred up and the diluent is added in the test tube. After recapping the tube, the tube is gently tilted a few times before putting in the refrigerator. The solution in the second step has to be heated up to the temperature of the sperm, which is in this case about room temperature, thus the bottle is put long enough in advance at room temperature (about 15 minutes for 100 ml starting with a solution at 37°C, or 30 minutes for 100 ml starting with a solution at 5°C).

The invention also relates to a method to conserve fresh sperm comprising the following steps:
a) preparing or obtaining a liquid composition as defined above,
b) optionally, making powder of said composition for the long term conservation of said composition at room temperature for an unlimited time, preferably using a freeze-drying procedure, or obtaining said powder composition,
c) optionally, dissolving said powder using water, preferably demineralised or sterile water,
d) optionally, storing said liquid composition at about 4°C for maximum 7 days
e) stabilizing said composition for about 30 min at 18 to 25°C,
f) warming up the composition to 37°C for about 15 min for 100 ml,
g) optionally, filtering the sperm, preferably using a gauze,
h) mixing the composition of step f) with the sperm, optionally of step g), preferably until a sperm cell concentration between about 20-60 X10⁶ cells/ml, preferably of 40X10⁶ cells/ml is reached,
i) decreasing the temperature slowly at a rate of 0.2 -0.5°C per minute until about 5°C is reached, and,
j) conserving said composition at about 5°C in an anaerobic condition.

The invention also relates to a method to fertilize oocytes of animal or human origin, comprising following steps:
a) preparing or obtaining a liquid composition as defined above,
b) optionally, making powder of said composition for the long term conservation of said composition at room temperature for an unlimited time, preferably using a freeze-drying procedure, or obtaining said powder composition,
c) optionally, dissolving said powder using water, preferably demineralised or sterile water,
d) optionally, storing said liquid composition at about 4°C for maximum 7 days,
e) stabilizing said composition for about 30 min at 18 to 25°C,
f) warming up the composition to 37°C for about 15 min for 100 ml,
g) optionally, filtering the sperm, preferably using a gauze,
h) mixing the composition of step f) with the sperm, optionally of step g), preferably until a sperm cell concentration between about 20-60 X10⁶ cells/ml, preferably of 40X10⁶ cells/ml is reached,
i) decreasing the temperature slowly at a rate of 0.2 -0.5°C per minute until about 5°C is reached,
j) conserving said composition at about 5°C in an anaerobic condition.
k) optionally conserving said composition at about 5°C in an anaerobic condition,
I) optionally, warming said composition to RT/37°C, and,
m) using said composition for the fertilization of oocytes.

According to a further embodiment said fertilization is performed in vitro or in vivo.

The invention also relates to a vial comprising a liquid composition as defined above or a powder composition as defined above.

According to a further embodiment said vial comprises enough liquid or powder to make a solution required to conserve sperm necessary for at least one oocyte fertilization.

According to a further embodiment said vial is made out of glass or plastic.

The invention also relates to a method to prepare a vial as defined above comprising following steps:
a) preparing a liquid composition as defined above,
b) optionally, making powder of said composition for the long term conservation of said composition preferably using a freeze-drying procedure, and,
c) adding said composition according to step a) or b) into a suitable vial.

### DETAILED DESCRIPTION

Milk has effective qualities in its use as a thinner. It is a buffered environment and contains components that offer protection to spermatozoids, such as fats, proteins and lactose.

The thinner described in the present invention is meant for internal application. It is thus essential that one use a sanitary or purified and safe solution.

This thinner offers a relative satisfaction. The thinner is easier to prepare and clear of aspect. A method for sterilization of the solution after preparation is not necessary, if the preparation of the thinner is carried out in optimal circumstances in order to avoid contamination.

If desired, the thinner can be used without adding any antibiotics to the environment as these can all have an effect on the motility and the fertility of the sperm.

Sterilization of the thinner before adding the sperm by means of membrane filtration may be considered.

Long term storage of thinner can occur in small quantities at -20°C. The freeze-drying of the thinner of the present invention offers some interesting benefits. The chances of contamination have been reduced to a minimum since the customer dissolves only the required quantity just prior to use.

The required volume of thinner may also be made up fresh every day, at least a half hour in advance, in order to be certain that it is a homogeneous buffered solution. The excess quantity of the solution can be stored in the fridge for at least 7 days, on the condition that the solution has not been heated up. The excess quantity of thinner that has been warmed up cannot be used thereafter, except during the same day.

A further advantage of the thinner in freeze-dried conditions is the almost unlimited life cycle.

The thinner of the present invention offers the possibility to optimize the sperm production. This means making up a significant number of dosages containing a minimum quantity of spermatozoids.

The lactose and glucose quantities in the liquid thinner composition as defined above can be adapted. The motility duration of the sperm is primarily dependent on these quantities.

Additional sterilization of the thinner is not necessary when starting with sterile water and sterilized recipients. The liquid thinner solution as defined above may be immediately stored at 5°C and the required quantities may be warmed up prior to use. The remaining solution can be preserved for 7 days in the fridge without problems.

As with all buffers, the thinner of the present invention needs at least 30 minutes to stabilize prior to its use.

### FIGURE LEGENDS

### Figure 1.

Comparison of different thinners as set out in Example 1 (Ghentse thinner (Allmed), Ovusper thinner (Virbac) and the thinner of the present invention termed "Zangersheide" thinner) for the stallions Campary Z (A), Zandor Z (B) and Ulrich Z (C).

In said comparison the % linearity greater than 40% is set out for different times of incubation of the sperm in said thinner. Linearity (LIN) equals VSL/VCL. VSL refers to Straight-line velocity and VCL refers to Curvilinear velocity. Curvilinear velocity (VCL) is characterized as the centroid-to-centroid path, and velocity along that path. Straight-line velocity (VSL) is characterized as the linear path between the first and last centroid in a sequence , and velocity along that path.

### Figure 2.

Comparison of different thinners as set out in Example 1 (Ghentse thinner (Allmed), Ovusper thinner (Virbac) and the thinner of the present invention termed "Zangerheide" thinner) for the stallions Campary Z (A), Zandor Z (B) and Ulrich Z (C).

In said comparison the % straightness greater than 50% is set out for different times of incubation of the sperm in said thinner. Straightness (STR) equals VSL/VAP. VSL refers to Straight-line velocity and VAP refers to Average path velocity. Average path velocity (VAP) is characterized as the smoothed centroid-to-centroid path, as calculated by one of several smoothing algorithms, and velocity along that path. Straight-line velocity (VSL) is characterized as the linear path between the first and last centroid in a sequence , and velocity along that path.

### Figure 3.

Comparison of different thinners as set out in Example 1 (Ghentse thinner (Allmed), Ovusper thinner (Virbac) and the thinner of the present invention termed "Zangersheide" thinner) for the stallions Ulrich Z (A), Zandor Z (B) and Campary Z (C).

In said comparison the % of quick cells (having a VSL of more than 30µm/s) is set out for different times of incubation of the sperm in said thinner. Straight-line velocity (VSL) is characterized as the linear path between the first and last centroid in a sequence , and velocity along that path.

### EXAMPLES

### Example 1. Preparation of the thinners used in the comparative study

The thinners used for comparison to the thinner of the present invention are purchased thinners.

Ovusper distributes via VIRBAC thinner for equine sperm based on egg yolk. Ghentse thinner, based on milk en egg yolk, is made up in the RUG and is distributed by Allmed.

The following thinner composition according to the present invention was used.

| | |
|---|---|
| Glucose monohydrate | 27.2 g ; |
| Lactose monohydrate | 1.5 g , |
| Sodium citrate dihydrate | 0.25 g , |
| Potassium citrate | 0.41 g , |
| Hepes | 4.75 g , |
| Non-fat powdered milk | 50 g , |
| optionally, antibiotics | 50 mg , and, water. |

### Example 2. Effect of the different thinners on the motility of the mobility of the spermatozoids in vitro

Five studs were used in a comparative study with at least 3 stallions per experiment. After collection, the sperm was filtered through a gauze and thinned to a concentration of 40x10⁶ cells per ml in the different thinners tested out. Subsequently, all conditions are kept at 5°C in an anaerobic environment.

The analysis of the sample was carried out following a 5-minute heating at 37°C. The quality of the sperm is evaluated by means of an automatic analysis of the motility of the spermatozoids (Hamilton Thorn Motility Analyser IVOS). The quick spermatozoids are kept as criteria. This means those with a velocity greater than 30µm/s.

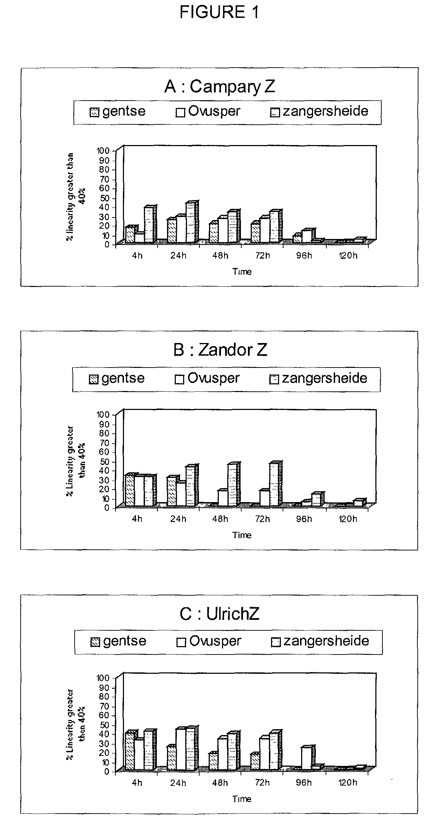

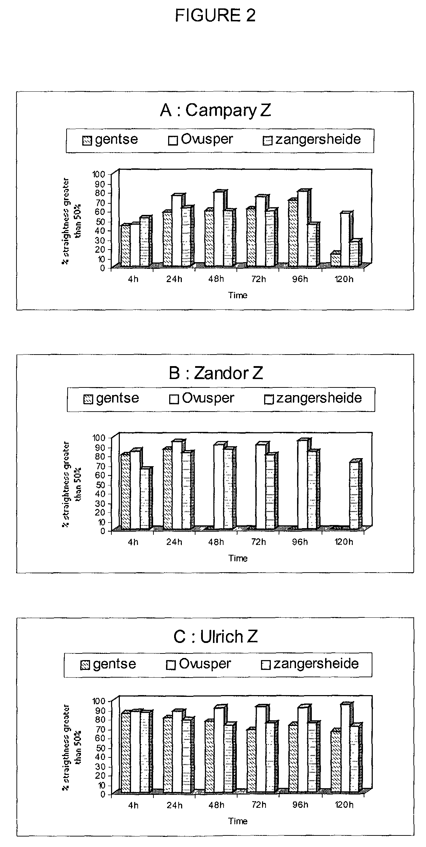

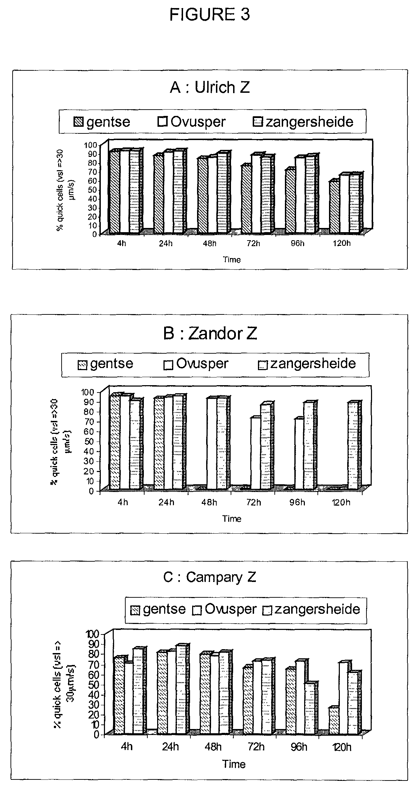

The results of the comparative experiment are shown in Figures 1 to 3.

In the prior art there is no question of the straightness of the sperm. At first sight, the thinner of the present invention displays a better straightness than the other thinners. The straightness may be a more important feature than the speed, as it is of utmost important that the sperm cells get where they are needed, at the oocyte, rather than the speed in which they reach their target.

### Example 3. Effect of the different thinners on the fertility in vitro

This experiment of testing in vivo is performed with insemination every 48 hours, starting with a follicle of 33mm until the ovulation. The concentration dosage used in the experiment is only 1/3 of the normal dosage used.

## Claims

1. A liquid composition comprising per liter:
| | |
|---|---|
| Glucose monohydrate | 0.1-50 g ; |
| Lactose monohydrate | 0.1-50 g , |
| Sodium citrate dihydrate | 0.1-1 g , |
| Potassium citrate | 0.1-1 g , |
| Hepes | 0.1-10 g , |
| Non-fat powdered milk | 40-60 g , |
| optionally, antibiotics | 10-100 mg , and, |
| water. | |

2. A liquid composition according to claim 1 comprising per liter:
| | |
|---|---|
| Glucose monohydrate | 27.2 g ; |
| Lactose monohydrate | 1.5 g , |
| Sodium citrate dihydrate | 0.25 g , |
| Potassium citrate | 0.41 g , |
| Hepes | 4.75 g , |
| Non-fat powdered milk | 50 g , |
| optionally, antibiotics | 50 mg , and, |
| water. | |

3. A liquid composition according to claim 1 or 2, wherein said milk is Gloria® manufactured by Nestle®.

4. A liquid composition according to any of claims 1 to 3, wherein said water is demineralised; and preferably sterilized.

5. A liquid composition according to any of claims 1 to 4, wherein said antibiotic is Gentamycin.

6. A liquid composition according to any of claims 1 to 5, wherein said liquid composition has a pH between 6.3 and 7.0.

7. A powder composition prepared by means of a drying procedure starting from a composition of any of claims 1 to 5.

8. A powder composition according to claim 7, wherein said drying procedure is a freeze-drying procedure.

9. A powder composition according to claim 7 or 8, wherein said composition is stored in the dark at temperatures between about 15 and 25°C for an unlimited time.

10. A liquid composition prepared by dissolving a powder composition according to any of claims 7 to 9, using water; preferably demineralised or sterilized water.

11. A liquid composition according to any of claims 1 to 6 and 10, wherein said composition is stored at about 5°C.

12. A liquid composition according to any of claims 1 to 6 and 10 to 11, wherein said composition is stabilized at room temperature for at least about 30 min and heated to about 37°C just before use.

13. A liquid composition according to any of claims 1 to 6 and 10 to 12, wherein said composition is mixed with sperm.

14. A liquid composition according to claim 13, wherein said mixing is performed by swirling the recipient at the first stage of the processing of the sperm, after the doses are prepared, using a slowly rotating roller bank.

15. A liquid composition according to claim 13 or 14, wherein said sperm is of animal or human origin.

16. A liquid composition according to claim 15, wherein said animal is chosen from the group sheep, cattle, pig and horse.

17. A liquid composition according to any of claim 13 to 16, wherein said sperm is filtered before mixing it with the liquid composition according to any of claims 1 to 6 and 10 to 12.

18. A liquid composition according to claim 17, wherein said filter filtration is performed using a gauze.

19. A liquid composition according to any of claims 1 to 6 and 10 to 12, wherein said liquid composition is sterilized using an UHT method.

20. A liquid composition according to any of claims 13 to 19 wherein said sperm is diluted into the composition according to any of claims 1 to 6 and 10 to 12 reaching a sperm cell concentration between about 20-60x10⁶ cells/ml, preferably of 40x10⁶ cells/ml.

21. A liquid composition according to claim 20, wherein said composition is used immediately to perform fertilization of oocytes, or, is conserved between about 2-10°C, preferably at about 5°C in anaerobic condition.

22. Use of a composition according to any of claims 1 to 12 as a sperm thinner.

23. Use of a composition according to any of claims 1 to 21, for the conservation of fresh sperm.

24. Use of a composition according to any of claims 13 to 21, for the fertilization of oocytes.

25. A method for the preparation of a thinner for the conservation of fresh sperm, comprising following steps:
a) preparing a liquid composition according to any of claims 1 to 6, and,
b) optionally, making powder of said composition for the long term conservation of said dried composition preferably using a freeze-drying procedure.

26. A method for the preparation of a sperm solution for the fertilization of oocytes, comprising following steps:
a) preparing or obtaining a liquid composition according to any of claims 1 to 6,
b) optionally, making a powder of said composition for the long term conservation of said composition at room temperature for an unlimited time, preferably using a freeze-drying procedure, or obtaining said powder composition,
c) optionally, dissolving said powder using water, preferably demineralised or sterile water,
d) optionally, storing said liquid composition at about 4°C for maximum 7 days,
e) stabilizing said composition for about 30 min at 18 to 25°C,
f) warming up the composition to 37°C for about 15 min for 100 ml,
g) optionally, filtering the sperm, preferably using a gauze,
h) mixing the composition of step f) with the sperm, optionally of step g), preferably until a sperm cell concentration between about 20-60x10⁶ cells/ml, preferably of 40x10⁶ cells/ml is reached,
i) decreasing the temperature slowly at a rate of 0.2-0.5°C per minute until about 5°C is reached, and,
j) conserving said composition at about 5°C in an anaerobic condition.

27. A method to conserve fresh sperm comprising the following steps:
a) preparing or obtaining a liquid composition according to any of claims 1 to 6,
b) optionally, making powder of said composition for the long term conservation of said composition at room temperature for an unlimited time, preferably using a freeze-drying procedure, or obtaining said powder composition,
c) optionally, dissolving said powder using water, preferably demineralised or sterile water,
d) optionally, storing said liquid composition at about 4°C for maximum 7 days
e) stabilizing said composition for about 30 min at 18 to 25°C,
f) warming up the composition to 37°C for about 15 min for 100 ml,
g) optionally, filtering the sperm, preferably using a gauze,
h) mixing the composition of step f) with the sperm, optionally of step g), preferably until a sperm cell concentration between about 20-60x10⁶ cells/ml, preferably of 40x10⁶ cells/ml is reached,
i) decreasing the temperature slowly at a rate of 0.2-0.5°C per minute until about 5°C is reached, and,
j) conserving said composition at about 5°C in an anaerobic condition.

28. A method to fertilize oocytes, comprising following steps:
a) preparing or obtaining a liquid composition according to any of claims 1 to 6,
b) optionally, making powder of said composition for the long term conservation of said composition at room temperature for an unlimited time, preferably using a freeze-drying procedure, or obtaining said powder composition,
c) optionally, dissolving said powder using water, preferably demineralised or sterile water,
d) optionally, storing said liquid composition at about 4°C for maximum 7 days,
e) stabilizing said composition for about 30 min at 18 to 25°C,
f) warming up the composition to 37°C for about 15 min for 100 ml,
g) optionally, filtering the sperm, preferably using a gauze,
h) mixing the composition of step f) with the sperm, optionally of step g), preferably until a sperm cell concentration between about 20-60x10⁶ cells/ml, preferably of 40x10⁶ cells/ml is reached,
i) decreasing the temperature slowly at a rate of 0.2-0.5°C per minute until about 5°C is reached,
j) conserving said composition at about 5°C in an anaerobic condition.
k) optionally conserving said composition at about 5°C in an anaerobic condition,
I) optionally, warming said composition to RT/37°C, and,
m) using said composition for the fertilization of oocytes.

29. A method according to claim 28, whereby said fertilization is performed in vitro or in vivo.

30. A vial comprising a liquid composition according to any of claims 1 to 6 and 10 to 12, or, a powder composition according to any of claims 8 to 9.

31. A vial according to claim 30, comprising enough liquid or powder to make a solution required to conserve sperm necessary for at least one oocyte fertilization.

32. A vial according to claim 30 or 31, which is made out of glass or plastic.

33. A method to prepare a vial according any of claims 30 to 32, comprising following steps:
a) preparing a liquid composition according to any of claims 1 to 6,
b) optionally, making powder of said composition for the long term conservation of said composition preferably using a freeze-drying procedure, and,
c) adding said composition according to step a) or b) into a suitable vial.
